# EUROPÄISCHE PATENTANMELDUNG

(11) **EP 4 349 399 A1**
(43) Veröffentlichungstag der Anmeldung: **10.04.2024**
(21) Anmeldenummer: 22200323.8
(22) Anmeldetag: 07.10.2022
(51) Int. Cl.: A61N 5/06

(54) **INFRAROTKABINE MIT EINEM SYSTEM ZUM EINSTELLEN EINER THERMONEUTRALZONE EINES NUTZERS**

(71) Anmelder: Mattheiss, Gerd, 7203 Trimmis (CH)
(72) Erfinder: Pecher, Otto, 85653 Aying b. München (DE); Zeiger, Thomas, 6134 Vomp (AT); Mattheiss, Gerd, 7203 Trimmis (CH)
(74) Vertreter: Fuchs Patentanwälte Partnerschaft mbB

(57) **Zusammenfassung**

Die vorliegende Erfindung betrifft eine Infrarotkabine zur Wärmebestrahlung eines Nutzers, umfassend ein Sitz- und/oder Liegemöbel, einen in das Sitz- und/oder Liegemöbel integrierten Infrarotstrahler, und ein System zum Einstellen einer Thermoneutralzone des sich auf dem Sitzund/oder Liegemöbel befindenden Nutzers der Infrarotkabine.

## Beschreibung

Die vorliegende Erfindung betrifft eine Infrarotkabine zur Wärmebestrahlung eines Nutzers, umfassend ein Sitz- und/oder Liegemöbel, einen in das Sitz- und/oder Liegemöbel integrierten Infrarotstrahler, und ein System zum Einstellen einer Thermoneutralzone des sich auf dem Sitz- und/oder Liegemöbel befindenden Nutzers der Infrarotkabine.

### Stand der Technik

Wärmebehandlungsvorrichtungen sind bekannt. Sie werden üblicherweise dazu verwendet, Wärme in Form von Wärme- bzw. Infrarotstrahlung auf den Körper oder bestimmte Körperbereiche eines Nutzers aufzubringen. Eine Wärmebehandlung geschieht oft als therapeutische oder therapiebegleitende Maßnahme, etwa im Rahmen physiotherapeutischer Behandlungen, oder allgemein zur Förderung des Wohlbefindens.

Allerdings hat sich herausgestellt, dass abhängig von dem Nutzer auf herkömmliche Weise durchgeführte Wärmeanwendungen nicht immer zu optimalen Ergebnissen führen.

Es ist daher eine Aufgabe der vorliegenden Erfindung, Mittel anzugeben, mit denen die Nachteile des Stands der Technik überwunden werden können und die insbesondere eine wirksame und/oder individualisierte Wärmeanwendung ermöglichen.

### Beschreibung der Erfindung

Die Aufgabe wird gelöst durch eine Infrarotkabine gemäß dem unabhängigen Anspruch.

Gemäß einem **ersten Aspekt** der Erfindung ist eine Infrarotkabine zur Wärmebestrahlung eines Nutzers vorgesehen, die ein Sitz- und/oder Liegemöbel, einen in das Sitz- und/oder Liegemöbel integrierten Infrarotstrahler, und ein System zum Einstellen einer (der) Thermoneutralzone des sich auf dem Sitz- und/oder Liegemöbel befindenden Nutzers der Infrarotkabine umfasst. Der in das Sitz- und/oder Liegemöbel integrierte Infrarotstrahler dient zur eigentlichen Wärmebehandlung des Nutzers und kann insbesondere dazu ausgebildet sein, einen Rückenbereich des Nutzers zu bestrahlen.

Das System umfasst wenigstens einen Sensor zum Messen zumindest einer Messgröße, vorzugsweise am Ort einer Körperoberfläche des Nutzers, und wenigstens einen Thermoneutralzonenwärmestrahler. Insbesondere kann der wenigstens eine Sensor so angeordnet und eingerichtet sein, eine Messgröße in einem Bereich der Infrarotkabine zu messen, der nicht oder zumindest nicht direkt von dem in das Sitz- und/oder Liegemöbel integrierten Strahler angestrahlt wird. Der Thermoneutralzonenstrahler umfasst vorzugsweise einen Infrarotstrahler. Das System weist eine Kontrolleinheit auf, die dazu eingerichtet ist, basierend auf einem Ergebnis einer Auswertung eines mit dem wenigstens einen Sensor aufgenommenen Messwertes eine Steuerung und/oder Regelung des Thermoneutralzonenwärmestrahlers zum Einstellen der Thermoneutralzone vorzunehmen. Genauer gesagt kann die Kontrolleinheit nach Maßgabe des Ergebnisses der Auswertung des mit dem wenigstens einen Sensor aufgenommenen Messwertes den Thermoneutralzonenwärmestrahler so steuern und/oder regeln, dass dieser zum einen die den Nutzer umgebende Luft in der Kabine erwärmt, insbesondere in einem den Nutzer unmittelbar umgebenden Bereich, und zum anderen Wärme an den Nutzer mittels Infrarotstrahlung abgibt bzw. mittels Infrarotstrahlung Wärme in den Nutzer einbringt. Zusammen kann der Thermoneutralzonenwärmestrahler so die Thermoneutralzone des Nutzers einstellen.

Der Erfindung liegt die überraschende Erkenntnis zugrunde, dass eine Wärmeanwendung besonders vorteilhaft bei einem Nutzer im Sinne einer schonenden Durchwärmung des gesamten Körpers wirken kann, wenn sich der Nutzer in seiner Thermoneutralzone befindet. Indem erfindungsgemäß ein Thermoneutralzonenwärmestrahler nach Maßgabe von Messwerten in Bezug auf den Nutzer geregelt bzw. gesteuert wird, können die Umgebungsbedingungen des Nutzers und damit die Rahmenbedingungen vor und während einer durchzuführenden Wärmeanwendung auf den Nutzer zuverlässig eingestellt werden. Dadurch kann mit anderen Worten vorteilhaft eine an den Nutzer individuell angepasste Thermoneutralzone eingestellt werden. Dies wiederum begünstigt ein Wohlbefinden des Nutzers während der Anwendung und eine vorteilhafte Wirkungsentfaltung der Wärmeanwendung im Sinne einer schonenden Durchwärmung.

Die Messgröße kann eine (Umgebungs-)Lufttemperatur in der Infrarotkabine sein, insbesondere eine (Umgebungs-)Lufttemperatur in einem den Nutzer unmittelbar umgebenden Bereich. Der wenigstens eine Sensor kann einen Temperatursensor umfassen oder ein Temperatursensor sein. Alternativ oder zusätzlich kann die Messgröße eine Infrarotstrahlungsstärke des Thermoneutralzonenwärmestrahlers sein, insbesondere eine an den Nutzer abgegebene oder in den Nutzer eingebrachte Infrarotstrahlungsstärke bzw. Wärmestrahlungsstärke sein. Der wenigstens eine Sensor kann einen IR-Sensor und/oder eine Wärmebildkamera umfassen oder ein IR-Sensor und/oder eine Wärmebildkamera sein. Alternativ oder zusätzlich kann die Messgröße eine relative Luftfeuchtigkeit in der Infrarotkabine sein, insbesondere in einem den Nutzer unmittelbar umgebenden Bereich. Der wenigstens eine Sensor kann einen Luftfeuchtigkeitssensor umfassen oder ein Luftfeuchtigkeitssensor sein.

Alternativ oder zusätzlich handelt es sich bei der Messgröße um einen Vitalparameter des Nutzers. Beispielsweise kann dies der Puls, eine lokale Temperatur (beispielsweise eine Hauttemperatur, wie eine Temperatur auf einer Hautoberfläche und/oder innerhalb von Hautschichten) und/oder ein Hautwiderstand jeweils des Nutzers sein. Die Messgröße kann eine Wärmeabbaurate des Nutzers sein.

Beispielsweise kann es sich bei der Messgröße auch um einen Parameter der Umgebung des Nutzers handeln, wie etwa eine Strahlungsintensität, eine Luftfeuchtigkeit, eine Lufttemperatur und/oder eine Windgeschwindigkeit.

Ein exemplarischer Sensor des zumindest einen Sensors kann auch mehr als eine Messgröße am Ort des Körpers des Nutzers messen.

Die Kontrolleinheit kann die Messwerte unter Berücksichtigung eines Korrekturfaktors verarbeiten. Hierbei kann unter anderem die Position des Thermoneutralzonenstrahlers und/oder des wenigstens einen Sensors relativ zum Nutzer und/oder relativ zueinander berücksichtigt werden.

Neben dem Thermoneutralzonenstrahler können auch weitere Faktoren und Einrichtungen die Lufttemperatur in der Infrarotkabine und somit die Einstellung der Thermoneutralzone beeinflussen. So kann beispielsweise der in das Sitz- und/oder Liegemöbel integrierte Infrarotstrahler und/oder ein Radiatorgebläse Wärme an die Luft abgeben und die Lufttemperatur so erhöhen. Auch dies kann von der Kontrolleinheit berücksichtigt werden, da diese Beeinflussung ebenfalls mittels des wenigstens einen Sensors erfasst wird. Entsprechendes gilt für weitere Faktoren, wie zum Beispiel die äußere Umgebungstemperatur um die Infrarotkabine herum und/oder weitere Wärmequellen innerhalb oder außerhalb der Infrarotkabine.

Die Infrarotkabine kann ein Kabinengehäuse umfassen, in dem die spezifizierten Bestandteile der Infrarotkabine angeordnet sein können. Die Infrarotkabine kann für den Nutzer durch eine in dem Kabinengehäuse vorgesehene Tür begehbar sein. Das Kabinengehäuse kann vorwiegend aus Holz bestehen und/oder Holz umfassen. Alternativ kann die Infrarotkabine eine Zeltkonstruktion sein.

Die Infrarotkabine kann beispielsweise eine Sitzkabine und/oder eine Liegekabine sein.

Die Auswertung des mit dem Sensor aufgenommenen Messwertes kann in einer Auswerteeinheit der Infrarotkabine, insbesondere des Systems, erfolgen. Die Auswerteeinheit kann räumlich-körperlich in die Infrarotkabine integriert oder in einer mit dem System verbundenen Cloud vorgesehen sein. Alternativ kann die Auswerteeinheit eine externe Auswerteeinheit sein, die das Ergebnis an die Infrarotkabine liefert. Beispielsweise kann die Auswerteeinheit mittels WLAN, Bluetooth und/oder anderen Funkstandards mit den weiteren Systemkomponenten verbunden sein.

In einer Ausführungsform kann der zumindest eine Sensor direkt an dem Nutzer angeordnet oder anordenbar sein. Alternativ kann der zumindest eine Sensor auch beabstandet von dem Nutzer vorgesehen sein. Insbesondere Sensoren zur Messung der Hauttemperatur sind bevorzugt zur berührungslosen Messwertaufnahme ausgelegt und daher beabstandet zu dem Nutzer angeordnet oder anordenbar.

In einer Ausführungsform kann die Kontrolleinheit dazu eingerichtet sein, Messwerte von dem zumindest einen Sensor zu empfangen und/oder auszuwerten.

In einer Ausführungsform kann das Steuern und/oder Regeln des wenigstens einen Thermoneutralzonenwärmestrahlers aufweisen, ein Kontrollsignal zu erzeugen, das dem wenigstens einen Thermoneutralzonenwärmestrahler zugeführt oder zuführbar ist, und/oder eine Strahlungsintensität des wenigstens einen Thermoneutralzonenwärmestrahlers anzupassen, insbesondere zu erhöhen oder zu verringern.

Im Sinne der vorliegenden Anmeldung wird vorzugsweise unter einer Thermoneutralzone (TNZ) eine oder mehrere Umgebungsbedingungen, insbesondere ein Bereich von Umgebungstemperaturen, des Nutzers verstanden, unter der oder denen der Nutzer seine normale Körperinnen- oder Körperkerntemperatur mit minimaler Stoffwechselregulierung aufrechterhalten kann. Jeder Nutzer hat eine individuelle Thermoneutralzone. Mit anderen Worten kann ein Nutzer in seiner Thermoneutralzone seine normale Körperinnentemperatur aufrechterhalten, ohne über den normalen Grundumsatz hinaus Energie verbrauchen zu müssen, d.h. ohne dass der Nutzer dafür Wärme (beispielsweise durch Zittern und/oder regulieren der Hautdurchblutung) produzieren muss oder dass er Wärme (beispielsweise durch Schwitzen mit einhergehender Verdunstungskühlung) abführen muss. Eine Thermoneutralzone ist für jeden Nutzer individuell und von einer Vielzahl nutzerbezogener Faktoren abhängig, z.B. Körpergewicht, Körperfettanteil, Geschlecht, individuelles Befinden. Innerhalb der Thermoneutralzone kann die Grundrate der Wärmeproduktion gleich der Rate der Wärmeabgabe an die Umgebung sein. Die Thermoneutralzone kann bei unbekleideten normalgewichtigen Männern bei etwa 27 °C beginnen und kann bis etwa 35 °C Umgebungstemperaturen reichen. Die Thermoneutralzone kann bei unbekleideten normalgewichtigen Damen bei etwa 30 °C beginnen und kann bis etwa 37 °C Umgebungstemperaturen reichen.

In einer Ausführungsform kann vorgesehen sein, dass der wenigstens eine Thermoneutralzonenwärmestrahler eine Wärmequelle, beispielsweise in Form eines Dunkelstrahlers (also eines Infrarotstrahlers mit einer Verblendung aus Spezialkeramik oder Metall) oder eines Halogenstrahlers, vorzugsweise einen internen Reflektor, und ein Gehäuse aufweist, wobei vorzugsweise die Wärmequelle und der interne Reflektor zumindest bereichsweise von dem Gehäuse umhaust und/oder zumindest teilweise innerhalb des Gehäuses angeordnet sind. Es sind auch Ausbildungen eines Thermoneutralzonenwärmestrahlers ohne internen Reflektor möglich.

Der Thermoneutralzonenwärmestrahler ist also vorteilhaft mehrteilig aufgebaut. Insbesondere ein interner Reflektor kann dazu vorgesehen sein, die von der Wärmequelle erzeugte Wärmestrahlung in Richtung des Nutzers umzulenken. Dadurch kann beispielsweise die Wärmequelle vor einer Berührung geschützt innerhalb des Gehäuses, etwa in einem Seitenbereich desselbigen, angeordnet sein. Die von der Wärmequelle abgestrahlte Wärmestrahlung kann dann über den internen Reflektor nach außerhalb des Gehäuses zu dem Nutzer reflektiert werden.

Das Gehäuse kann, insbesondere an der Innenseite, zumindest bereichsweise eine Wärmeisolierung aufweisen. Dadurch kann eine Erwärmung des Gehäuses vermieden oder zumindest reduziert werden und somit ein sicherer Betrieb erreicht werden. Auch kann das Material des Gehäuses und des internen Reflektors so gewählt sein, dass eine Erwärmung des Gehäuses minimiert wird.

In einer Ausführungsform kann vorgesehen sein, dass an dem Gehäuse, insbesondere an einem oder mehreren Seitenbereichen und/oder einer Gehäuseöffnung, ein Schutzgitter und/oder eine Glasabdeckung vorgesehen ist oder sind.

Beispielsweise kann das Schutzgitter eine Öffnung des Gehäuses abdecken. Dazu, aber auch in allen anderen Fällen, ist das Schutzgitter vorzugsweise lösbar mit dem Gehäuse verbunden. Dadurch kann das Schutzgitter von dem Gehäuse abgenommen werden, um beispielsweise einen Innenbereich des Gehäuses zu erreichen und diesen zu reinigen oder darin angeordnete Komponenten auszutauschen.

Beispielsweise kann die Glasabdeckung eine Öffnung des Gehäuses abdecken. Dazu, aber auch in allen anderen Fällen, ist die Glasabdeckung vorzugsweise lösbar mit dem Gehäuse verbunden. Dadurch kann die Glasabdeckung von dem Gehäuse abgenommen werden, um beispielsweise einen Innenbereich des Gehäuses zu erreichen und diesen zu reinigen oder darin angeordnete Komponenten auszutauschen.

Der wenigstens eine Thermoneutralzonenwärmestrahler kann eine, insbesondere maximale, Abstrahltemperatur von wenigstens 250° C haben, vorzugsweise von wenigstens 800° C. In einer Ausführungsform kann der wenigstens eine Thermoneutralzonenwärmestrahler eine, insbesondere maximale, Abstrahltemperatur zwischen 250° C und 400 °C haben.

Die Abstrahltemperatur des wenigstens einen Thermoneutralzonenwärmestrahlers hat Einfluss auf bzw. entscheidet über die spektrale Verteilung. Die spektrale Verteilung des wenigstens einen Thermoneutralzonenwärmestrahlers kann somit über die Veränderung der Leistung des wenigstens einen Thermoneutralzonenwärmestrahlers in gewünschter Weise variiert werden.

In einer Ausführungsform kann vorgesehen sein, dass der wenigstens eine Thermoneutralzonenwärmestrahler ein Flächen-Strahler, ein Dunkel-Strahler, insbesondere aus eloxiertem Aluminium, ein offener Strahler und/oder ein 3D-Strahler ist oder umfasst. Ein 3D-Strahler kann ein ergonomisch, dreidimensional geformter Strahler sein.

In einer Ausführungsform kann der wenigstens eine Thermoneutralzonenwärmestrahler eine Wärmequelle und einen internen Reflektor aufweisen. Die Wärmequelle kann wenigstens ein Heizstab sein, vorzugsweise eine Heizwendel oder ein Keramik-/lncoloystab. Ein solcher Thermoneutralzonenwärmestrahler mit Heizstab weist eine vergleichsweise geringe Abstrahlfläche auf, die sich durch die Mantelfläche des Heizstabs definiert. Der Heizstab kann einen Durchmesser von 10 mm aufweisen. Der wenigstens eine Thermoneutralzonenwärmestrahler kann eine, insbesondere maximale, Abstrahltemperatur von wenigstens 800° C haben, insbesondere unter Verwendung eines Halogenstabs als Wärmequelle. Der wenigstens eine Thermoneutralzonenwärmestrahler kann eine, insbesondere maximale, Abstrahltemperatur von wenigstens 250° C haben, insbesondere unter Verwendung eines Keramik- oder Incoloystabs als Wärmequelle.

In einer Ausführungsform kann der wenigstens eine Thermoneutralzonenwärmestrahler eine Wärmequelle aufweisen und ohne internen Reflektor ausgebildet sein. Die Wärmequelle kann einen Flächenstrahler mit Keramik und/oder einer beschichteten Materialoberfläche umfassen. Ein solcher Thermoneutralzonenwärmestrahler mit Flächenstrahler weist eine vergleichsweise große Abstrahlfläche auf. Die Abstrahlfläche kann wenigstens 500 mm x 100 mm betragen. Der wenigstens eine Thermoneutralzonenwärmestrahler kann eine, insbesondere maximale, Abstrahltemperatur von weniger als 400° C haben, vorzugsweise zwischen 250° C und 400° C.

Unter einem offenen Strahler wird vorzugsweise ein Strahler verstanden, dessen Wärmequelle auch während des regulären Betriebs des Thermoneutralzonenwärmestrahlers von außen, insbesondere frontal, zugänglich ist und sich insbesondere nicht hinter einer Abdeckung befindet.

In einer Ausführungsform kann vorgesehen sein, dass der wenigstens eine Thermoneutralzonenwärmestrahler mehrere Teil-Wärmestrahler aufweist, welche vorzugsweise zumindest teilweise unabhängig voneinander aktivierbar und/oder deaktivierbar sind, insbesondere manuell und/oder durch die dazu entsprechend eingerichtete Kontrolleinheit.

Jeder Teil-Wärmestrahler kann vorzugsweise die Merkmale aufweisen, wie sie zuvor in Bezug auf den Wärmestrahler beschrieben worden sind und zwar einzeln und in beliebiger Kombination.

Der Thermoneutralzonenwärmestrahler kann vorteilhaft als eine Gruppe von Teil-Wärmestrahlern ausgebaut sein. Beispielsweise können alle oder einige Teil-Wärmestrahler in einem gemeinsamen Gehäuse untergebracht sein. Die Teil-Wärmestrahler können zum Einstellen der Thermoneutralzone zusammenwirken.

Alternativ oder ergänzend kann vorgesehen sein, dass der wenigstens eine Thermoneutralzonenwärmestrahler eine Sitzheizung umfasst. Diese kann in das Sitz-/Liegemöbel integriert sein. Die Sitzheizung kann insbesondere einen Teil-Wärmestrahler darstellen. Dadurch können besonders zielgerichtet und über den Körper des Nutzers hinweg in variabler Ausgestaltung die Umgebungsbedingungen des Nutzers beeinflusst werden. Bei vergleichsweise kalten Kabinen kann eine Sitzheizung hilfreich sein, um möglichst schnell die Thermoneutralzone einzustellen.

In einer Ausführungsform weist das Sitz- und/oder Liegemöbel ein oder mehrere Schweißauffang- und/oder -Schweißsammelmittel zum Auffangen und/oder Sammeln von Schweiß des Nutzers auf. Beispielsweise kann somit Schweiß, der in den Wärmestrahler tropft aufgefangen und gesammelt werden. Beispielsweise lässt sich der Schweiß so nachträglich analysieren. Dies kann auch einen vollkommen eigenständigen Aspekt des Sitz- und/oder Liegemöbels für eine Infrarotanwendung darstellen, unabhängig von dem Thermoneutralzonenwärmestrahler und anderen Komponenten der Infrarotkabine.

In einer Ausführungsform kann vorgesehen sein, dass das System zumindest zwei Sensoren zum Messen jeweils zumindest einer Messgröße in der Infrarotkabine und/oder am Ort der Körperoberfläche des Nutzers aufweist.

Durch den Einsatz mehrerer Sensoren können mehr Informationen in Bezug auf den Nutzer (etwa Vitalparameter des Nutzers) und/oder dessen Umgebung erfasst werden. Dies ermöglicht es, den Thermoneutralzonenwärmestrahler kontrollierter zu regeln bzw. zu steuern und dadurch die Thermoneutralzone definierter und individualisierter einzustellen.

In einer Ausführungsform sind die Sensoren der zumindest zwei Sensoren von unterschiedlichem Sensortyp.

Die Sensoren oder einer der Sensoren können Temperatursensoren sein. Die Sensoren oder einer der Sensoren können Sensoren zur Messung der Infrarotstrahlungsstärke sein, beispielsweise Infrarotsensoren oder Wärmebildkameras oder sonstige Sensoren mit Photozelle. Die Sensoren oder einer der Sensoren können Luftfeuchtigkeitssensoren sein.

In einer Ausführungsform kann vorgesehen sein, dass ein erster Sensor des zumindest einen Sensors eine Temperatur, wie eine Lufttemperatur eines Umgebungsbereichs des Nutzers und/oder eine Hauttemperatur des Nutzers misst.

Dazu ist der erste Sensor während des Einsatzes des Systems vorteilhafterweise derart angeordnet oder anordenbar, dass der Erfassungsbereich des ersten Sensors während des Einsatzes des Systems zur Erfassung eines bestimmten Hautbereichs entsprechend ausgerichtet ist.

Alternativ oder ergänzend kann vorgesehen sein, dass ein zweiter Sensor des zumindest einen Sensors eine Bestrahlungsstärke (Infrarotbestrahlungsstärke) an der Körperoberfläche, insbesondere der Hautoberfläche, des Nutzers oder in einem zu dieser Körperoberfläche beabstandeten Flächen- und/oder Volumenbereich misst.

Dazu ist der zweite Sensor während des Einsatzes des Systems vorteilhafterweise derart angeordnet oder anordenbar, dass der Erfassungsbereich des zweiten Sensors während des Einsatzes des Systems zur Erfassung der Hautoberfläche, des Flächenbereichs und/oder des Volumenbereichs entsprechend ausgerichtet ist.

In einer Ausführungsform sind die Sensoren der zumindest zwei Sensoren von unterschiedlichen Sensortypen, wobei vorzugsweise jeder der Sensoren eine Messgröße in Bezug auf die Umgebung des Nutzers in der Infrarotkabine oder in Bezug auf den wenigstens einen Thermoneutralzonenstrahler aufnimmt. Beispielsweise kann zumindest ein Sensor als ein Temperatursensor ausgebildet sein und die Lufttemperatur im Umgebungsbereich des Nutzers messen. Der zweite Sensor kann ein Infrarotsensor oder eine Wärmebildkamera sein und die Intensität der Wärmestrahlung des wenigstens einen Thermoneutralzonenstrahlers messen.

In einer Ausführungsform sind die Sensoren der zumindest zwei Sensoren vom gleichen Sensortyp, wobei jeder der Sensoren eine Messgröße in Bezug auf die Umgebung des Nutzers in der Infrarotkabine oder in Bezug auf einen Körperbereich des Nutzers aufnimmt. Beispielsweise können zumindest zwei Sensoren jeweils als ein Temperatursensor ausgebildet sein und mit einem ersten Temperatursensor ist Lufttemperatur im Umgebungsbereich des Nutzers und mit dem zweiten Temperatursensor ist eine Hauttemperatur am Körper des Nutzers messbar.

In einer Ausführungsform sind die Sensoren der zumindest zwei Sensoren vom gleichen Sensortyp, wobei die einzelnen Sensoren Messgrößen in Bezug auf verschiedene Körperbereiche des Nutzers aufnehmen. Beispielsweise können zumindest zwei Sensoren jeweils als ein Temperatursensor ausgebildet sein und mit einem ersten Temperatursensor ist eine Hauttemperatur im Gesicht und mit dem zweiten Temperatursensor ist eine Hauttemperatur im Nackenbereich des Nutzers messbar. Dazu sind die Sensoren entsprechend angeordnet, dass sich während des Einsatzes des Systems der entsprechend positionierte Nutzer mit Gesicht und Nacken im Erfassungsbereich der jeweiligen Sensoren befindet.

In einer Ausführungsform kann vorgesehen sein, dass die Kontrolleinheit dazu eingerichtet ist, anhand der Sensordaten, insbesondere der mit dem ersten Sensor gemessenen Temperatur und/oder der mit dem zweiten Sensor gemessenen Bestrahlungsstärke, den aufgrund des wenigstens einen Thermoneutralzonenwärmestrahlers zumindest bereichsweise verursachten Wärmeeintrag auf die Körperoberfläche des Nutzers oder ein Maß dafür zu ermitteln und diesen bei der Steuerung und/oder Regelung des wenigstens einen Thermoneutralzonenwärmestrahlers einzubeziehen.

Unter Berücksichtigung des Wärmeeintrags, vorzugsweise und anderer vorgegebener und/oder gemessener Parameter, kann besonders zuverlässig ermittelt werden, ob sich der Nutzer in seiner individuellen Thermoneutralzone befindet. Daher kann nach Maßgabe dieser/s Parameter/s sehr zuverlässig der Thermoneutralzonenwärmestrahler kontrolliert, insbesondere dessen Wärmeabgabe verringert oder erhöht, werden.

Beispielsweise können vor Beginn einer Anwendung weitere Parameter vom Nutzer oder einem Betreiber der Infrarotkabine dem System zur Verfügung gestellt werden. Diese Parameter können nutzerbezogene Daten wie Geschlecht, Körpergröße und -gewicht, tagesaktuelles Wohlbefinden, Gesundheitszustand, etc. umfassen.

Bei der Auswertung können die aufgenommenen Messwerte mit den weiteren Parametern in Relation gesetzt und verarbeitet werden.

Abhängig von der Auswertung kann die Kontrolleinheit beispielsweise die Intensität des Wärmestrahlers erhöhen. Anderenfalls kann die Kontrolleinheit die Intensität des Wärmestrahlers vorteilhafterweise reduzieren.

In einer Ausführungsform kann vorgesehen sein, dass die Kontrolleinheit dazu eingerichtet ist, bei der Steuerung und/oder Regelung des wenigstens einen Thermoneutralzonenwärmestrahlers auf den Nutzer bezogene Daten, welche die Kontrolleinheit vorzugsweise von einem Eingabemittel, wie einem mobilen Endgerät, direkt oder indirekt empfängt, einzubeziehen.

Nutzerbezogene Daten können beispielsweise ein oder mehrere der folgenden Daten aufweisen: Ein Wohlfühlmaß des Nutzers, das Körpergewicht des Nutzers, die Körpergröße des Nutzers, das Alter des Nutzers, ein Stoffwechselfaktor des Nutzers, eine Körperoberfläche des Nutzersoder ein Maß dafür und/oder eine Maßzahl für die Effizienz der Thermoregulation.

Beispielsweise kann das System und/oder die Kontrolleinheit mit einem mobilen Endgerät, zum Beispiel einem Smartphone, koppelbar oder gekoppelt sein, beispielsweise über eine Funkverbindung. Dann kann die Kontrolleinheit dazu eingerichtet sein, nutzerbezogene Daten von dem mobilen Endgerät zu empfangen.

Beispielsweise kann das System und/oder die Kontrolleinheit mit einer zentralen Recheneinheit, zum Beispiel über eine Cloudanwendung, koppelbar oder gekoppelt sein, beispielsweise über eine Internetverbindung. Dann kann die Kontrolleinheit dazu eingerichtet sein, nutzerbezogene Daten von der zentralen Recheneinheit zu empfangen.

In einer Ausführungsform kann vorgesehen sein, dass das System ferner einen externen Reflektor zum Reflektieren von zumindest einem Teil der von dem wenigstens einen Thermoneutralzonenwärmestrahler abgegebenen Wärmestrahlung in Richtung des Nutzers aufweist, wobei vorzugsweise (i) der externe Reflektor und der wenigstens eine Thermoneutralzonenwärmestrahler derart zueinander angeordnet sind, dass der wenigstens eine Thermoneutralzonenwärmestrahler zumindest einen Teil der Wärmestrahlung in Richtung des externen Reflektors abstrahlt, (ii) der externe Reflektor und der wenigstens eine Thermoneutralzonenwärmestrahler als separate Module ausgebildet sind und/oder (iii) beim Einsatz des Systems der externe Reflektor und der wenigstens eine Thermoneutralzonenwärmestrahler einen Abstand zueinander von wenigstens 0,5 m, vorzugsweise von wenigstens 1,0 m, aufweisen.

Durch den externen Reflektor sind Bereiche des Nutzers mit Wärmestrahlung beaufschlagbar, welche sich von dem Thermoneutralzonenwärmestrahler aus gesehen nicht in direkter Sichtlinie befinden.

Beispielsweise kann mittels eines an einer Seitenwand befestigten externen Reflektors Wärmestrahlung, die von einem an einer Decke montierten Thermoneutralzonenwärmestrahler ausgestrahlt wird, in Richtung einer Vorderseite (anterior, bauchseitig) eines sitzenden Nutzers reflektiert werden. Dadurch kann also der Körper des Nutzers vorderseitig zumindest abschnittsweise mit Wärmestrahlung beaufschlagt werden, obwohl sich dieser Bereich von dem Thermoneutralzonenwärmestrahler aus gesehen zumindest teilweise nicht in direkter Sichtlinie befindet und/oder zum Beispiel von dem Kopf des Nutzers zumindest teilweise abgeschattet wird.

Daher ist vorteilhafterweise der externe Reflektor derart relativ zu dem Thermoneutralzonenwärmestrahler angeordnet, dass während des Einsatzes des Systems Bereiche des Nutzers mit Wärmestrahlung beaufschlagbar sind, welche sich von dem Thermoneutralzonenwärmestrahler aus gesehen zumindest teilweise nicht in direkter Sichtlinie befinden und/oder von anderen Teilen des Körpers des Nutzers zumindest teilweise abgeschattet sind.

Der oder die externe/n Reflektor/en kann ein unangenhmes Gefühl auf der Hautoberfläche des Nutzers durch kühle Seitenwandbereiche (insbesondere Glaswände/Glasabschnitte) der Kabine reduzieren. Beispielsweise haben Glaswände höhere Wärmeverluste nach außen und werden daher vom Nutzer, wenn nah am Nutzer platziert, als kühl/unangenehm empfunden. Dem kann mittels des oder der externe/n Reflektor/en entgegengewirkt werden.

Der Mindestabstand zwischen dem externen Reflektor und dem Thermoneutralzonenwärmestrahler bezeichnet vorzugsweise den kleinsten Abstand zwischen diesen beiden Komponenten, der zwischen zwei Punkten der beiden Komponenten messbar ist.

In einer Ausführungsform kann das Sitz- und/oder Liegemöbel ein als Sitz- und/oder Liegemöbel nutzbares Möbel sein. Das Sitz- und/oder Liegemöbel kann lösbar innerhalb der Infrarotkabine anordenbar sein.

Die Infrarotkabine kann mehr als ein Sitz- und/oder Liegemöbel umfassen, beispielsweise für Paar- oder Gruppenanwendungen.

In einer Ausführungsform kann das Sitzmöbel in ein Liegemöbel überführbar sein und umgekehrt.

Die Infrarotkabine, insbesondere das Sitz- und/oder Liegemöbel, kann eine Kopfstütze zum Abstützen des Kopfes des Nutzers aufweisen. Optional ist die Kopfstütze mit der Sitz- und/oder Liegegelegenheit bereitgestellt und/oder einstückig ausgebildet.

In einer Ausführungsform kann vorgesehen sein, dass der erste Sensor in und/oder an einer von dem Sitz- und/oder Liegemöbel aufgewiesenen Kopfstütze zum Abstützen des Kopfes des Nutzers vorgesehen ist.

Dadurch kann besonders vorteilhaft ein Bereich des Nackens und/oder des Kopfes des Nutzers mit dem ersten Sensor erfassbar sein und somit die dort bestehende Temperatur aufgenommen werden.

Beispielsweise kann in der vorgeschlagenen Konfiguration mittels des Temperatursensor eine Hauttemperatur eines Nackenbereiches des Nutzers erfassbar sein.

In einer Ausführungsform kann vorgesehen sein, dass der wenigstens eine Thermoneutralzonenwärmestrahler an, über oder in der Decke der Infrarotkabine und/oder an, unter oder in dem Boden der Infrarotkabine angeordnet ist und/oder der externe Reflektor an, hinter oder in einer Seitenwand der Kabine angeordnet ist. Insbesondere kann die Infrarotkabine in diesem Fall eine Sitzkabine oder zumindest zeitweise in einer Konfiguration als Sitzkabine betreibbar sein.

Durch den externen Reflektor sind Bereiche des Nutzers mit Wärmestrahlung beaufschlagbar, welche sich von dem Wärmestrahler aus gesehen nicht in direkter Sichtlinie befinden.

Beispielsweise kann mittels eines an einer Seitenwand befestigten externen Reflektors Wärmestrahlung, die von einem an einer Decke befestigten Thermoneutralzonenwärmestrahler ausgestrahlt wird, in Richtung einer Vorderseite (anterior, bauchseitig) eines sitzenden Nutzers reflektiert werden. Dadurch kann also der Körper des Nutzers vorderseitig zumindest abschnittsweise mit Wärmestrahlung beaufschlagt werden, obwohl sich dieser Bereich von dem Thermoneutralzonenwärmestrahler aus gesehen zumindest teilweise nicht in direkter Sichtlinie befindet und/oder beispielsweise von dem Kopf des Nutzers zumindest teilweise abgeschattet wird.

Daher ist vorteilhafterweise der externe Reflektor derart relativ zu dem Thermoneutralzonenwärmestrahler angeordnet, dass während des Einsatzes der Kabine Bereiche (wie Oberflächenbereiche) des Nutzers mit Wärmestrahlung durch Reflexion der von dem Thermoneutralzonenwärmestrahler auf den externen Reflektor auftreffenden Wärmestrahlung beaufschlagbar sind, welche Bereiche sich von dem Thermoneutralzonenwärmestrahler aus gesehen nicht in direkter Sichtlinie befinden und/oder von anderen Teilen des Körpers des Nutzers abgeschattet sind. Vorteilhafterweise ist die Abstrahlcharakteristik des Thermoneutralzonenwärmestrahler dabei derart, dass zumindest Teile der abgestrahlten Wärmestrahlung auf den externen Reflektor auftreffen. Somit kann der Wärmestrahler vor Berührung geschützt an der Decke montiert sein.

Alternativ oder ergänzend kann vorgesehen sein, dass der wenigstens eine Thermoneutralzonenwärmestrahler an, hinter oder in einer Seitenwand der Kabine angeordnet ist und/oder der externe Reflektor an, über oder in der Decke und/oder an, unter oder in dem Boden der Kabine angeordnet ist. Insbesondere kann die Infrarotkabine in diesem Fall eine Liegekabine ist oder zumindest zeitweise in einer Konfiguration als Liegekabine betreibbar sein.

Durch den externen Reflektor sind Bereiche des Nutzers mit Wärmestrahlung beaufschlagbar, welche sich von dem Thermoneutralzonenwärmestrahler aus gesehen nicht in direkter Sichtlinie befinden.

Beispielsweise kann mittels eines an einer Decke befestigten externen Reflektors Wärmestrahlung, die von einem an einer (z.B. hinteren oder vorderen) Seitenwand befestigten Thermoneutralzonenwärmestrahler ausgestrahlt wird, in Richtung einer Vorderseite (anterior, bauchseitig) eines liegenden Nutzers reflektiert werden. Dadurch kann also der Körper des Nutzers vorderseitig zumindest abschnittsweise mit Wärmestrahlung beaufschlagt werden, obwohl sich dieser Bereich von dem Thermoneutralzonenwärmestrahler aus gesehen zumindest teilweise nicht in direkter Sichtlinie befindet und/oder beispielsweise von dem Kopf des Nutzers zumindest teilweise abgeschattet wird.

Eine Seitenwand kann eine beliebige Innenseitenwand der Infrarotkabine beschreiben. Beispielsweise kann eine Seitenwand eine der vier Seitenwände (links, rechts, vorne, hinten) einer im Grundriss rechteckigen Kabine sein.

Daher ist vorteilhafterweise der externe Reflektor derart relativ zu dem Thermoneutralzonenwärmestrahler angeordnet, dass während des Einsatzes der Kabine Bereiche (wie Oberflächenbereiche) des Nutzers mit Wärmestrahlung durch Reflexion der von dem Thermoneutralzonenwärmestrahler auf den externen Reflektor auftreffenden Wärmestrahlung beaufschlagbar sind, welche Bereiche sich von dem Thermoneutralzonenwärmestrahler aus gesehen nicht in direkter Sichtlinie befinden und/oder von anderen Teilen des Körpers des Nutzers abgeschattet sind. Vorteilhafterweise ist die Abstrahlcharakteristik des Thermoneutralzonenwärmestrahler dabei derart, dass zumindest Teile der abgestrahlten Wärmestrahlung auf den externen Reflektor auftreffen. Dadurch kann auch ein schwerer Thermoneutralzonenwärmestrahler vorteilhaft an der Kabinenwand montiert sein und muss nicht mit zusätzlichen Sicherungen an der Decke befestigt sein.

In einer Ausführungsform kann vorgesehen sein, dass der wenigstens eine Thermoneutralzonenwärmestrahler und der externe Reflektor derart relativ zueinander angeordnet und derart ausgebildet sind, dass die von dem wenigstens einen Thermoneutralzonenwärmestrahler abgestrahlte Wärmestrahlung zumindest teilweise in Richtung des externen Reflektors abgestrahlt wird und zumindest teilweise an dem externen Reflektor in Richtung des Nutzers reflektiert wird.

Es versteht sich, dass die Infrarotkabine in einer Ausführungsform verschiedene Kombinationen von Thermoneutralzonenwärmestrahlern und Reflektoren aufweisen kann. So kann eine Infrarotkabine beispielsweise einen an der Decke angeordneten Thermoneutralzonenwärmestrahler und einen oder mehrere an Seitenwänden angeordnete Thermoneutralzonenwärmestrahler umfassen. Auch können zusätzlich oder alternativ externe Reflektoren der beschriebenen Art vorgesehen sein.

In einer Ausführungsform kann vorgesehen sein, dass die Infrarotkabine in einer ersten Konfiguration als Sitzkabine und in einer zweiten Konfiguration als Liegekabine betreibbar ist und wobei vorzugsweise (i) die Kontrolleinheit dazu eingerichtet ist, abhängig von der gewählten Konfiguration die Intensität der Wärmestrahlung, mit der der externe Reflektor beaufschlagt wird, anzupassen und/oder (ii) die Intensität der Wärmestrahlung, mit der der externe Reflektor beaufschlagt wird, anpassbar ist.

Indem die Infrarotkabine in mehreren Konfigurationen betreibbar ist, können zahlreiche Wärmebehandlungsarten und/oder vorzugsweise lokale Kältebehandlungen im Sinne einer Reiztherapie durchführbar sein. Dies macht die vorgeschlagene Kabine besonders flexibel einsetzbar.

Beispielsweise kann eine Rückenlehne eines Sitzmöbels abgenommen oder umgeklappt werden, um die Kabine von der einen Konfigurationen (Sitzkabine) in die andere Konfiguration (Liegekabine) zu überführen.

Beispielsweise kann auch ein Sitzmöbel durch ein Liegemöbel ausgetauscht werden, um die Kabine von der einen Konfigurationen (Sitzkabine) in die andere Konfiguration (Liegekabine) zu überführen. Dazu kann das Sitz- und Liegemöbel jeweils lösbar mit dem Boden der Kabine verbindbar sein.

Indem die Intensität der Strahlung, mit der der externe Reflektor beaufschlagt wird angepasst wird oder anpassbar ist, kann vorteilhaft mit einem einzigen Thermoneutralzonenwärmestrahler ein Betrieb der Infrarotkabine in beiden Konfigurationen zuverlässig ermöglicht werden. So kann in einer Konfiguration (etwa der ersten oder zweiten Konfiguration) beispielsweise sämtliche oder ein Großteil der Wärmestrahlung über den externen Reflektor auf den Nutzer gelenkt werden, während in einer anderen Konfiguration (etwa der zweiten oder ersten Konfiguration) beispielsweise sämtliche oder ein Großteil der Wärmestrahlung direkt (und insbesondere nicht über den externen Reflektor) auf den Nutzer gelenkt wird.

In einer Ausführungsform kann vorgesehen sein, dass die Kontrolleinheit dazu eingerichtet ist, abhängig von der gewählten Konfiguration (i) den wenigstens einen Thermoneutralzonenwärmestrahler mechanisch zwischen zumindest zwei Stellungen zu bewegen, insbesondere zu schwenken und/oder zu drehen, (ii) jeweils eine unterschiedliche Auswahl von Heizelementen des wenigstens einen Thermoneutralzonenwärmestrahlers zu aktivieren und/oder (iii) jeweils eine unterschiedliche Auswahl von Thermoneutralzonenwärmestrahlern und/oder Teil-Wärmestrahlern des wenigstens einen Thermoneutralzonenwärmestrahlers zu aktivieren.

Vorteilhafterweise erzeugen die unterschiedlichen Wärmequellen, Thermoneutralzonenwärmestrahler bzw. Teil-Wärmestrahler jeweils Wärmestrahlung in unterschiedliche Strahlungsrichtungen. Je nach Auswahl kann also beispielsweise eine direkte und indirekte Beaufschlagung des Nutzers mit Wärmestrahlung ermöglicht werden.

Auf diese Weise kann besonders zuverlässig zwischen einer direkten und indirekten Beaufschlagung des Nutzers mit Wärmestrahlung hin- und hergewechselt werden.

Es kann vorgesehen sein, dass der Thermoneutralzonenwärmestrahler manuell bewegbar, insbesondere schwenkbar und/oder drehbar ist, dass manuell eine unterschiedliche Auswahl von Wärmequellen des Thermoneutralzonenwärmestrahlers aktivierbar sind und/oder dass manuell eine unterschiedliche Auswahl von Thermoneutralzonenwärmestrahlern und/oder Teil-Wärmestrahlern des Thermoneutralzonenwärmestrahlers aktivierbar sind. Dies kann automatisiert und mittels der Kontrolleinheit gesteuert oder manuell etwa durch einen Bediener bewirkt werden.

Gemäß einem **zweiten Aspekt** ist eine Verwendung eines Thermoneutralzonenwärmestrahlers als Wärmestrahler in einem System der beschriebenen Art und/oder einer Infrarotkabine der beschriebenen Art vorgeschlagen.

Gemäß einem **dritten Aspekt** ist eine Verwendung eines Reflektors als externer Reflektor in einem System der beschriebenen Art und/oder einer Infrarotkabine der beschriebenen Art vorgeschlagen.

Gemäß einem **vierten Aspekt** ist eine Verwendung des Systems zum Einstellen einer individuellen Thermoneutralzone für einen Nutzer in einer Infrarotkabine und/oder im Rahmen einer Infrarotanwendung vorgesehen.

Alle Vorteile, Merkmale und Optionen, die in Bezug auf den ersten Aspekt der Erfindung beschrieben worden sind, gelten in Bezug auf den zweiten, dritten und vierten Aspekt der Erfindung entsprechend. Daher kann insoweit an dieser Stelle auf die vorherigen Ausführungen verwiesen werden.

### Kurzbeschreibung der Figuren

Weitere Merkmale und Vorteile der Erfindung ergeben sich aus der nachfolgenden Beschreibung, in der bevorzugte Ausführungsformen der Erfindung anhand schematischer Zeichnungen erläutert werden.

Dabei zeigen:
- Fig. 1: eine schematische Darstellung einer Infrarotkabine gemäß der Erfindung in einer ersten Ausführungsform;
- Fig. 2: eine schematische Darstellung einer Infrarotkabine gemäß der Erfindung in einer zweiten Ausführungsform;
- Fig. 3: eine schematische Darstellung einer Infrarotkabine gemäß der Erfindung in einer dritten Ausführungsform;
- Fig. 4: eine schematische Darstellung einer Infrarotkabine gemäß der Erfindung in einer vierten Ausführungsform; und
- Fig. 5: eine schematische Darstellung einer Infrarotkabine gemäß der Erfindung in einer fünften Ausführungsform.

### Beispiele

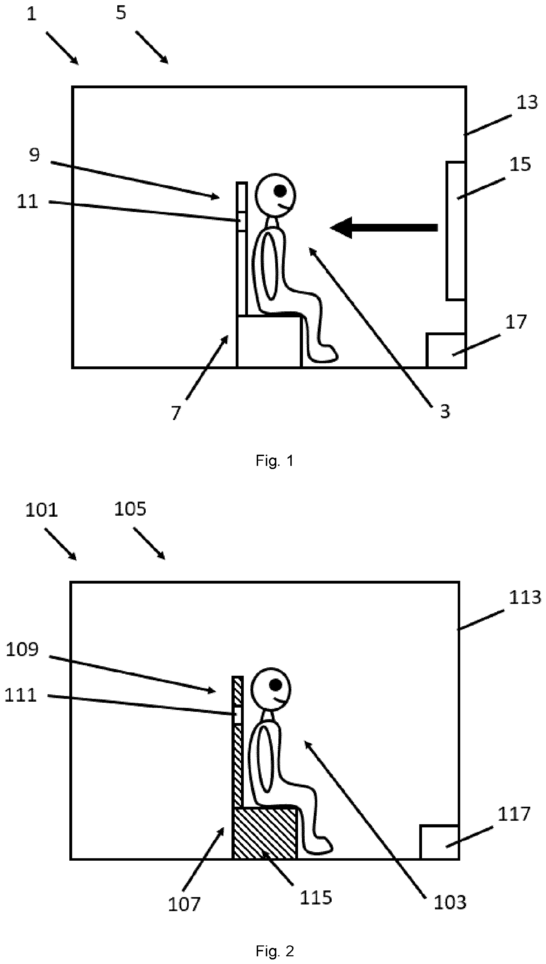

Figur 1 zeigt eine schematische Darstellung einer Kabine 1 in einer ersten Ausführungsform.

Bei der Kabine 1 handelt es sich um eine Wärmekabine, genauer gesagt um eine Infrarotkabine. Mit der Infrarotkabine 1 ist an einem Nutzer 3, hier ein Mensch, eine Wärmebehandlung durchführbar. Dazu weist die Infrarotkabine 1 ein System 5 zum Einstellen einer Thermoneutralzone für den Nutzer 3 auf.

In der Infrarotkabine 1 ist ein Sitzmöbel 7 vorgesehen. In der Kopfstütze 9 des Sitzmöbels 7 ist ein Sensor 11 des Systems 5 zum Messen einer Hauttemperatur des Nutzers 3 vorgesehen. An einer Seitenwand 13 der Infrarotkabine 1 ist ein Thermoneutralzonenwärmestrahler 15 des Systems 5 angeordnet. Der Thermoneutralzonenwärmestrahler 15 strahlt Wärmestrahlung vorwiegend (in Fig. 1) seitlich nach links ab und beaufschlagt den Nutzer 3 damit im Wesentlichen frontal von vorne, wie durch den dicken Pfeil angedeutet ist.

Außerdem weist das System 5 auch eine Kontrolleinheit 17 auf. Die Kontrolleinheit 17 wertet die mit dem Sensor 11 aufgenommenen Temperaturmesswerte der Hauttemperatur des Nutzers 3 aus und steuert und/oder regelt basierend auf einem Ergebnis der Auswertung den Thermoneutralzonenwärmestrahler 15, insbesondere indem er dessen Intensität anpasst. Dadurch kann zuverlässig die Thermoneutralzone des Nutzers 3 eingestellt werden. Auf diese Weise ist kannder Nutzer empfänglicher für eine Wärmeanwendung innerhalb der Infrarotkabine 1 sein. Für diese Wärmeanwendung kann die Infrarotkabine 1 weitere Mittel, wie mindestens einen Infrarotwärmestrahler, aufweisen, welche in Fig. 1 jedoch nicht dargestellt sind.

Figur 2 zeigt eine schematische Darstellung einer Infrarotkabine 101 gemäß einer zweiten Ausführungsform. Merkmale der Infrarotkabine 101, die gleich sind zu den Merkmalen der Infrarotkabine 1 sind mit gleichen, jedoch um 100 erhöhten Bezugszeichen versehen. Daher werden vorliegend nur die Unterschiede zwischen Infrarotkabine 1 und Infrarotkabine 101 erläutert.

Bei Infrarotkabine 101 ist der Thermoneutralzonenwärmestrahler 115 nicht an der Seitenwand 113, sondern innerhalb des Sitzmöbels 107 vorgesehen, wie durch die Schraffur angedeutet ist. Das heißt, der Thermoneutralzonenwärmestrahler 115 ist bei der Infrarotkabine 101 als Sitzheizung ausgebildet und erwärmt vorzugsweise neben der Sitzfläche auch die Rückenlehne einschließlich der Kopfstütze 109 des Sitzmöbels 107. Dadurch wird der Nutzer 103 bei Kontakt oder einem geringen Abstand mit dem Sitzmöbel 107 an den entsprechenden Körperregionen mit Wärme beaufschlagt.

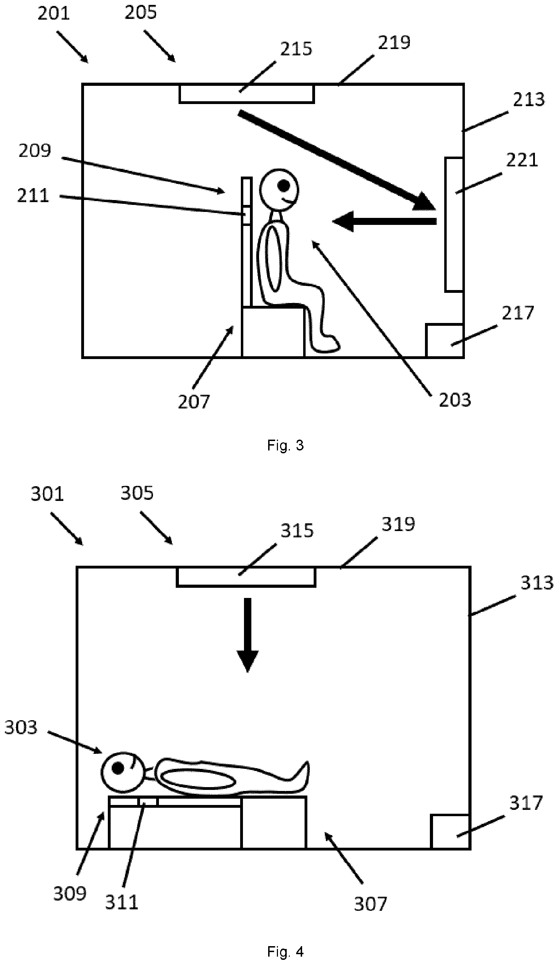

Figur 3 zeigt eine schematische Darstellung einer Infrarotkabine 201 gemäß einer dritten Ausführungsform. Merkmale der Infrarotkabine 201, die gleich sind zu den Merkmalen der Infrarotkabine 1 sind mit gleichen, jedoch um 200 erhöhten Bezugszeichen versehen. Daher werden vorliegend nur die Unterschiede zwischen Infrarotkabine 1 und Infrarotkabine 201 erläutert.

Bei Infrarotkabine 201 ist der Thermoneutralzonenwärmestrahler 215 nicht mehr an der Seitenwand 213, sondern an der Decke 219 der Infrarotkabine 201 angeordnet. Dort wo bei Infrarotkabine 1 der Thermoneutralzonenwärmestrahler 15 an der Seitenwand 213 vorgesehen war, ist bei Infrarotkabine 201 nun ein externer Reflektor 221 des Systems 205 angeordnet.

Der Thermoneutralzonenwärmestrahler 215 strahlt Wärmestrahlung hauptsächlich in Richtung des externen Reflektors 221, in Fig. 3 also nach schräg rechts unten, wie durch den dicken schrägen Pfeil angedeutet ist. Die auf den externen Reflektor 221 einfallende Wärmestrahlung wird von diesem in Richtung des Nutzers 203 reflektiert. Dadurch wird der Nutzer 203 im Ergebnis wieder mit aus Richtung der Seitenwand 213 bzw. des externen Reflektors 221 einfallenden Wärmestrahlung beaufschlagt, wie durch den dicken waagrechten Pfeil angedeutet ist. Allerdings kann bei der Infrarotkabine 201 der Thermoneutralzonenwärmestrahler 215 weitestgehend außerhalb der Reichweite des Nutzers 203 angeordnet und dadurch die Sicherheit beim Betrieb der Infrarotkabine 201 erhöht sein.

Damit ermöglicht es die Infrarotkabine 201, auch Körperbereiche des Nutzers 203 mit Wärme zu beaufschlagen, welche Bereiche durch andere Körperteile des Nutzers 203 abgeschattet werden (beispielsweise wird durch den Kopf der Brust- und Nackenbereich des Nutzers 203 ganz oder teilweise abgeschattet) und daher nicht in direkter Sichtlinie zu dem Thermoneutralzonenwärmestrahler 215 sind.

Figur 4 zeigt eine schematische Darstellung einer Infrarotkabine 301 gemäß einer vierten Ausführungsform. Merkmale der Infrarotkabine 301, die gleich sind zu den Merkmalen der Infrarotkabine 201 sind mit gleichen, jedoch um 100 erhöhten Bezugszeichen versehen. Daher werden vorliegend nur die Unterschiede zwischen Infrarotkabine 201 und Infrarotkabine 301 erläutert.

Bei Infrarotkabine 301 ist anstelle eines Sitzmöbels 207 nun ein Liegemöbel 307 vorgesehen. Im Gegensatz zu dem Thermoneutralzonenwärmestrahler 215, welcher eine Wärmeabstrahlung (in Fig. 3) vorwiegend nach schräg unten zur Seite hatte, strahlt der Thermoneutralzonenwärmestrahler 315 Wärmestrahlung vorwiegend direkt nach unten ab, wie durch den dicken Pfeil angedeutet ist. Dadurch wird der Nutzer 303 direkt von oben mit Wärmestrahlung beaufschlagt.

Ein externer Reflektor ist bei dem System 305 der Infrarotkabine 301 nicht vorgesehen. In einer Abwandlung der in Fig. 4 gezeigten vierten Ausführungsform könnte aber ein externer Reflektor am Platz des Thermoneutralzonenwärmestrahler 315 an der Decke 319 und der Thermoneutralzonenwärmestrahler 315 (wie bei der ersten in Bezug auf Fig. 1 beschriebenen Ausführungsform) an der Seitenwand 313 angeordnet sein. Indem der Thermoneutralzonenwärmestrahler 315 dann Wärmestrahlung vorwiegend nach schräg oben in Richtung des externen Reflektors abstrahlt, könnte der Nutzer 303 im Ergebnis ebenfalls mit von oben einfallender Wärmestrahlung beaufschlagt werden.

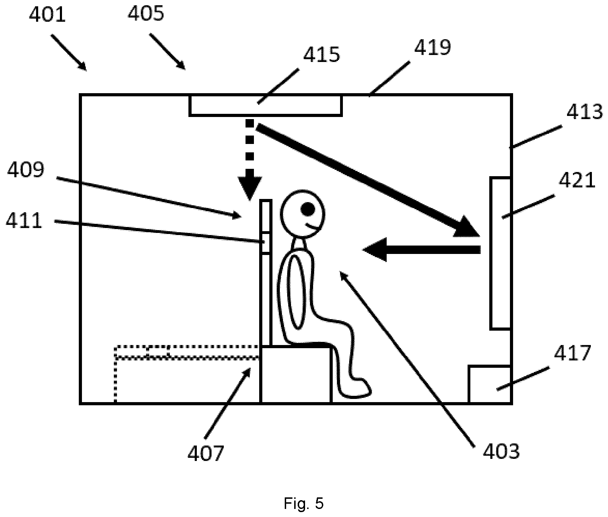

Figur 5 zeigt eine schematische Darstellung einer Infrarotkabine 401 gemäß einer fünften Ausführungsform. Merkmale der Infrarotkabine 401, die gleich sind zu den Merkmalen der Infrarotkabine 201 sind mit gleichen, jedoch um 200 erhöhten Bezugszeichen versehen. Daher werden vorliegend nur die Unterschiede zwischen Infrarotkabine 201 und Infrarotkabine 401 erläutert.

Die Infrarotkabine 401 kann in einer ersten Konfiguration als Sitzkabine betrieben werden und unterscheidet sich dann nicht von der Infrarotkabine 201. Die Infrarotkabine 401 kann ferner auch in einer zweiten Konfiguration als Liegekabine betrieben werden. Dazu kann die Rückenlehne des Möbels 407 umgeklappt werden, so dass sie die in Fig. 5 gestrichelt dargestellte Stellung einnimmt, welche vergleichbar mit der in Bezug auf Fig. 4 beschriebenen Situation ist.

Die Kontrolleinheit 417 ist dazu eingerichtet, abhängig von der gewählten Konfiguration die Intensität der Strahlung, mit der der externe Reflektor 421 beaufschlagt wird, anzupassen. Dazu ist die Kontrolleinheit 417 dazu eingerichtet, abhängig von der gewählten Konfiguration den Thermoneutralzonenwärmestrahler 415 mechanisch zwischen zwei Stellungen zu drehen. Je nach Ausrichtung des Thermoneutralzonenwärmestrahlers 415 strahlt dieser schräg zur Seite in Richtung des Reflektors 421 (das heißt, es liegt eine Nutzung der Infrarotkabine 401 als Sitzkabine ähnlich wie bei Infrarotkabine 201 vor und der Nutzer wird mit der Wärmestrahlung aus Richtung der Seitenwand 413 / des Reflektors 421 beaufschlagt, wie durch die dicken durchgezogenen Pfeile angedeutet ist) oder er strahlt direkt nach unten in Richtung des Nutzers 403 (das heißt, es liegt eine Nutzung der Infrarotkabine 401 als Liegekabine ähnlich wie bei Infrarotkabine 301 vor und der Nutzer 403 wird mit der Wärmestrahlung aus Richtung der Decke 419 / des Thermoneutralzonenwärmestrahlers 415 beaufschlagt, wie durch den dicken gestrichelten Pfeil angedeutet ist).

In einer Abwandlung erfolgt das Bewegen des Thermoneutralzonenwärmestrahlers 415 nicht durch die Kontrolleinheit 417, sondern ist manuell durch eine Person, etwa den Nutzer 403 selbst, durchführbar. In noch einer Abwandlung weist der Thermoneutralzonenwärmestrahler 415 mehrere Teil-Wärmestrahler auf. Einer der Teil-Wärmestrahler strahlt dann vorwiegend nach unten und ein anderer Teil-Wärmestrahler strahlt dann vorwiegend in Richtung des externen Reflektors 421 Wärmestrahlung ab. Je nach Konfiguration der Infrarotkabine 401 wird dann der eine oder andere Teil-Wärmestrahler manuell oder durch die Kontrolleinheit 417 aktiviert.

Die in der vorangehenden Beschreibung, in den Ansprüchen und in den Zeichnungen offenbarten Merkmale können sowohl einzeln als auch in beliebiger Kombination wesentlich für die Erfindung in ihren verschiedenen Ausführungsformen sein.

### Bezuqszeichenliste

- 1, 101, 201, 301, 401: Infrarotkabine
- 3,103,203,303,403: Nutzer
- 5, 105, 205, 305, 405: System
- 7, 107, 207, 307, 407: Möbel
- 9, 109, 209, 309, 409: Kopfstütze
- 11, 111, 211, 311, 411: Sensor
- 13, 113, 213, 313, 413: Seitenwand
- 15, 115, 215, 315, 415: Thermoneutralzonenwärmestrahler
- 17, 117, 217, 317, 417: Kontrolleinheit
- 219,319,419: Decke
- 221, 421: Externer Reflektor

## Patentansprüche

1. Infrarotkabine zur Wärmebestrahlung eines Nutzers, umfassend
ein Sitz- und/oder Liegemöbel;
einen in das Sitz- und/oder Liegemöbel integrierten Infrarotstrahler; und
ein System zum Einstellen einer Thermoneutralzone des sich auf dem Sitz- und/oder Liegemöbel befindenden Nutzers der Infrarotkabine, das wenigstens einen Sensor zum Messen zumindest einer Messgröße am Ort einer Körperoberfläche des Nutzers und wenigstens einen Thermoneutralzonenwärmestrahler umfasst, wobei das System eine Kontrolleinheit aufweist, die dazu eingerichtet ist, basierend auf einem Ergebnis einer Auswertung eines mit dem wenigstens einen Sensor aufgenommenen Messwertes eine Steuerung und/oder Regelung des Thermoneutralzonenwärmestrahlers zum Einstellen der Thermoneutralzone vorzunehmen.

2. Infrarotkabine nach Anspruch 1, wobei der wenigstens eine Thermoneutralzonenwärmestrahler eine Wärmequelle, vorzugsweise einen internen Reflektor, und ein Gehäuse aufweist, wobei vorzugsweise die Wärmequelle und der interne Reflektor zumindest bereichsweise von dem Gehäuse umhaust und/oder zumindest teilweise innerhalb des Gehäuses angeordnet sind.

3. Infrarotkabine nach Anspruch 2, wobei an dem Gehäuse, insbesondere an einem oder mehreren Seitenbereichen und/oder einer Gehäuseöffnung, ein Schutzgitter und/oder eine Glasabdeckung vorgesehen ist oder sind.

4. Infrarotkabine nach einem der vorangehenden Ansprüche, wobei der wenigstens eine Thermoneutralzonenwärmestrahler ein Flächen-Strahler, ein Dunkel-Strahler, insbesondere aus eloxiertem Aluminium, ein offener Strahler und/oder ein dreidimensional geformter Strahler ist.

5. Infrarotkabine nach einem der vorangehenden Ansprüche, wobei der wenigstens eine Thermoneutralzonenwärmestrahler mehrere Teil-Wärmestrahler aufweist, welche vorzugsweise zumindest teilweise unabhängig voneinander aktivierbar und/oder deaktivierbar sind, insbesondere manuell und/oder durch die dazu entsprechend eingerichtete Kontrolleinheit.

6. Infrarotkabine nach einem der vorangehenden Ansprüche, wobei der wenigstens eine Thermoneutralzonenwärmestrahler eine Sitzheizung umfasst.

7. Infrarotkabine nach einem der vorangehenden Ansprüche, wobei das System zumindest zwei Sensoren zum Messen jeweils zumindest einer Messgröße, insbesondere am Ort der Körperoberfläche des Nutzers, aufweist.

8. Infrarotkabine nach einem der vorangehenden Ansprüche, wobei ein erster Sensor des zumindest einen Sensors eine Temperatur, wie eine Lufttemperatur eines Umgebungsbereichs des Nutzers und/oder eine Hauttemperatur des Nutzers misst, und/oder
wobei ein zweiter Sensor des zumindest einen Sensors eine Bestrahlungsstärke an der Körperoberfläche, insbesondere der Hautoberfläche, des Nutzers oder in einem zu dieser Körperoberfläche beabstandeten Flächen- und/oder Volumenbereich misst.

9. Infrarotkabine nach einem der vorangehenden Ansprüche, wobei die Kontrolleinheit dazu eingerichtet ist, anhand der Sensordaten, insbesondere der mit dem ersten Sensor gemessenen Temperatur und/oder der mit dem zweiten Sensor gemessenen Bestrahlungsstärke, den aufgrund des wenigstens einen Thermoneutralzonenwärmestrahlers zumindest bereichsweise verursachten Wärmeeintrag auf die Körperoberfläche des Nutzers oder ein Maß dafür zu ermitteln und diesen bei der Steuerung und/oder Regelung des wenigstens einen Thermoneutralzonenwärmestrahlers einzubeziehen.

10. Infrarotkabine nach einem der vorangehenden Ansprüche, wobei die Kontrolleinheit dazu eingerichtet ist, bei der Steuerung und/oder Regelung des wenigstens einen Thermoneutralzonenwärmestrahlers auf den Nutzer bezogene Daten, welche die Kontrolleinheit vorzugsweise von einem Eingabemittel, wie einem mobilen Endgerät, direkt oder indirekt empfängt, einzubeziehen.

11. Infrarotkabine nach einem der vorangehenden Ansprüche, wobei das System ferner einen externen Reflektor zum Reflektieren von zumindest einem Teil der von dem wenigstens einen Thermoneutralzonenwärmestrahler abgegebenen Wärmestrahlung in Richtung des Nutzers aufweist, wobei vorzugsweise (i) der externe Reflektor und der wenigstens eine Thermoneutralzonenwärmestrahler derart zueinander angeordnet sind, dass der wenigstens eine Thermoneutralzonenwärmestrahler zumindest einen Teil der Wärmestrahlung in Richtung des externen Reflektors abstrahlt, (ii) der externe Reflektor und der wenigstens eine Thermoneutralzonenwärmestrahler als separate Module ausgebildet sind und/oder (iii) beim Einsatz des Systems der externe Reflektor und der wenigstens eine Thermoneutralzonenwärmestrahler einen Abstand zueinander von wenigstens 0,5 m, vorzugsweise von wenigstens 1,0 m, aufweisen.

12. Infrarotkabine nach einem der vorangehenden Ansprüche, wobei der wenigstens eine Thermoneutralzonenwärmestrahler an, über oder in der Decke der Infrarotkabine und/oder an, unter oder in dem Boden der Infrarotkabine angeordnet ist und/oder der externe Reflektor an, hinter oder in einer Seitenwand der Kabine angeordnet ist; und/oder
wobei der wenigstens eine Thermoneutralzonenwärmestrahler an, hinter oder in einer Seitenwand der Kabine angeordnet ist und/oder der externe Reflektor an, über oder in der Decke und/oder an, unter oder in dem Boden der Kabine angeordnet ist.

13. Infrarotkabine nach einem der vorangehenden Ansprüche, wobei der wenigstens eine Thermoneutralzonenwärmestrahler und der externe Reflektor derart relativ zueinander angeordnet und derart ausgebildet sind, dass die von dem wenigstens einen Thermoneutralzonenwärmestrahler abgestrahlte Wärmestrahlung zumindest teilweise in Richtung des externen Reflektors abgestrahlt wird und zumindest teilweise an dem externen Reflektor in Richtung des Nutzers reflektiert wird.

14. Infrarotkabine nach einem der vorangehenden Ansprüche, wobei die Infrarotkabine in einer ersten Konfiguration als Sitzkabine und in einer zweiten Konfiguration als Liegekabine betreibbar ist und wobei vorzugsweise (i) die Kontrolleinheit dazu eingerichtet ist, abhängig von der gewählten Konfiguration die Intensität der Wärmestrahlung, mit der der externe Reflektor beaufschlagt wird, anzupassen und/oder (ii) die Intensität der Wärmestrahlung, mit der der externe Reflektor beaufschlagt wird, anpassbar ist.

15. Infrarotkabine nach Anspruch 14, wobei die Kontrolleinheit dazu eingerichtet ist, abhängig von der gewählten Konfiguration (i) den wenigstens einen Thermoneutralzonenwärmestrahler mechanisch zwischen zumindest zwei Stellungen zu bewegen, insbesondere zu schwenken und/oder zu drehen, (ii) jeweils eine unterschiedliche Auswahl von Heizelementen des wenigstens einen Thermoneutralzonenwärmestrahlers zu aktivieren und/oder (iii) jeweils eine unterschiedliche Auswahl von Thermoneutralzonenwärmestrahlern und/oder Teil-Wärmestrahlern des wenigstens einen Thermoneutralzonenwärmestrahlers zu aktivieren.
